# EUROPEAN PATENT SPECIFICATION

(11) **EP 2 848 606 B1**
(45) Date of publication and mention of the grant of the patent: **30.08.2017**
(21) Application number: 13791641.7
(22) Date of filing: 07.05.2013
(51) Int. Cl.: C07C 279/16, A61K 31/155, A61P 31/16

(54) **FLUORO-SUBSTITUTED (3R, 4R, 5S)-5-GUANIDINO-4-ACYLAMINO-3-(PENTAN-3-YLOXY) CYCLOHEXENE-1-CARBOXYLIC ACIDS, ESTERS THEREOF AND A METHOD FOR THE USE THEREOF**
FLUORSUBSTITUIERTE (3R,4R,5S)-5-GUANIDINO-4-ACYLAMINO-3-(PENTAN-3-YLOXY)CYCLOHEXEN-1-CARBONSÄUREN, ESTER DAVON UND VERFAHREN ZUR VERWENDUNG DAVON
ACIDES (3R,4R,5S)-5-GUANIDINO-4-ACYLAMINO-3-(PENTAN-3-YLOXY) CYCLOHEXÈNE-1-CARBONIQUES SUBSTITUÉS EN FLUOR, LEURS ÉTHERS, ET PROCÉDÉ D'UTILISATION

(30) Priority: 12.05.2012 RU 2012119272
(43) Date of publication of application: 18.03.2015
(73) Proprietor: Ivachtchenko, Alexandre Vasilevich, Moscow Region Dolgoprudny 141700 (RU); Asavi, LLC, Hallandale Beach, Florida 33009 (US)
(72) Inventor: IVACHTCHENKO, Alexandre Vasilevich, Moscow Region Dolgoprudny 141700 (RU)
(74) Representative: Zellentin & Partner mbB Patentanwälte
(86) International application number: PCT/RU2013/000384
(87) International publication number: WO 2013/172741

(56) References cited:
- US-A- 5 952 375
- ZHENG MINGYUE ET AL.: 'QSAR analyses on avian influenza virus neuraminidase inhibitors using CoMFA, CoMSIA, and HQSAR.' JOURNAL OF COMPUTER-AIDED MOLECULAR DESIGN vol. 20, no. 9, 2006, pages 549 - 566, XP019450536 & DATABASE CA STN Database accession no. 945757-32-4
- DU OI-SHI ET AL.: 'Fragment-based quantitative structure-activity relationship (FB-QSAR) for fragment-based drug design.' JOURNAL OF COMPUTATIONAL CHEMISTRY vol. 30, no. 2, 2009, pages 295 - 304, XP055165717 & DATABASE CA STN Database accession no. 945757-32-4
- A. V. IVACHTCHENKO ET AL: "Novel oral anti-influenza drug candidate AV5080", JOURNAL OF ANTIMICROBIAL CHEMOTHERAPY., vol. 69, no. 7, 1 July 2014 (2014-07-01), pages 1892-1902, XP055297104, GB ISSN: 0305-7453, DOI: 10.1093/jac/dku074

## Description

### Field of invention

The present invention relates to novel compounds - fluorosubstituted (3R,4R,5S)-5-guanidino-4-acetamido-3-(pentan-3-yloxy)cyclohexene-1-carboxylic acids and their esters - inhibitors of neuraminidase activity.

### Background of the invention

Many microorganisms comprising neuraminidase are pathogenic to humans and animals such as poultry, horses, pigs and seals. These pathogenic microorganisms include influenza virus. Neuraminidase is associated with pathogenicity of influenza virus.

In this connection novel fluorosubstituted (3R,4R,5S)-5-guanidino-4-acetamido-3-(pentan-3-yloxy)cyclohexene-1-carboxylic acids and their esters are of primary concern as drug substances for medicaments intended for prophylaxis and treatment of influenza. The known neuraminidase inhibitors are (3R,4R,5S)-4-acetamido-5-amino-3-alkyloxy-cyclohexene-1-carboxylic acids **A1,** at that, the most active of them is (3R,4R,5S)-4-acetamido-5-amino-3-(pentan-3-yloxy)cyclohexene-1-carboxylic acid **A2,** which, as it was shown by X-ray diffraction data for its complex with neuraminidase of influenza virus, is effectively bound to the active center of the enzyme (Oseltamivir Carboxylate) [C. U. Kim, W. Lew, M. A. Williams, et al. J. Am. Chem. Soc. 1997, 119, 681-690.].

Ethyl ester of Oseltamivir carboxylic acid **A3,** known as Oseltamivir Phosphate or Tamiflu (Oseltamivir Phosphate, Tamiflu) [J. C. Rohloff, K. M. Kent, M. J. Postich, et al. J. Org. Chem. 1998, 63, 4545.], is a medicinal precursor of Oseltamivir carboxylate **A2.**

It is also known (3R,4R,5S)-5-guanidino-4-trifluoroacetamido-3-(pentan-3-yloxy)cyclohexene-1-carboxylic acid **A4,** exhibiting activity towards neuraminidase of influenza virus H5N1 [Q.-S. Du, R.-B. Huang, Y.-T. Wei, Z.-W. Pa, L.-Q. Du, K.-C. Chou. Fragment-Based Quantitative Structure-Activity Relationship (FB-QSAR) for Fragment-Based Drug Design. J. Comput. Chem. 2008, 30(2), 295-304].

(2*R*,3*R*,4*S*)-3-Acetamido-4-guanidino-2-((1*R*,2*R*)-1,2,3-trihydroxypropyl)-3,4-dihydro-2*H*-pyran-6-carboxylic acid (zanamivir) is known to be active against neuraminidase of influenza viruses A and B, and also neuraminidase of influenza virus H1 N1 [J. M. Woods, R. C. Bethell, J. A. Coates, et al. 4-Guanidino-2,4-dideoxy-2,3-dehydro-N-acetylneuraminic acid is a highly effective inhibitor both of the sialidase (neuraminidase) and of growth of a wide range of influenza A and B viruses in vitro. Antimicrob Agents Chemother. 1993, 37(7), 1473-1479].

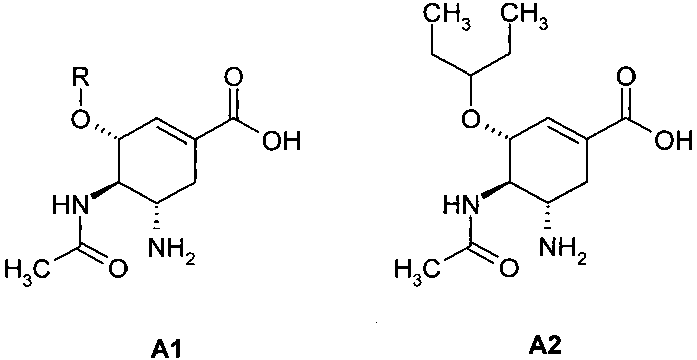

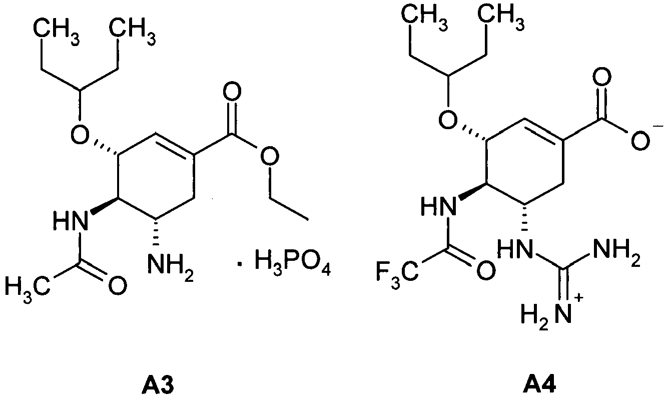

The US 5952375 A describes 5-guanidino-4-acylamino-3-alkoxycyclohexene-1-carboxylic acid, alkyl esters and salts thereof as neuraminidase inhibitors.

A study of biological properties concerning the general structural element of 5-guanidino-4-acylamino-3-alkoxycyclohexene-1-carboxylic acid, wherein the acylamino group is N(H)C(O)CF3 is described in ZHENG MINGYUE ET AL., "QSAR analyses on avian influenza virus neuraminidase inhibitors using CoMFA, CoMSIA, and HQSAR.", JOURNAL OF COMPUTER-AIDED MOLECULAR DESIGN, (2006), vol. 20, no. 9, pages 549 - 566.

Searching for highly effective anti-influenza medicaments, among other things exhibiting enhanced activity towards resistant influenza viruses, still remains one of the main trends in developing of novel pharmacological remedies for treating influenza. In this context, the design of novel anti-influenza ingredients, pharmaceutical compositions and medicaments, as well as methods for their preparation and use is of current interest.

### Disclosure of the invention

In context of the invention, terms are generally defined as follows: **"Alkyl"** means an aliphatic hydrocarbon straight or branched chain with 1-12 carbon atoms. Branched means the alkyl chain with at least one or more "lower alkyl" substituents. The preferred alkyl groups are methyl, trifluoromethyl, cyclopropylmethyl, cyclopentylmethyl, ethyl, *n*-propyl, *iso*-propyl, *n*-butyl, *tert*-butyl, *n*-pentyl, 3-pentyl, methoxyethyl, carboxymethyl, methoxycarbonylmethyl, ethoxycarbonylmethyl, benzyloxycarbonylmethyl and pyridylmethyloxycarbonylmethyl.
**"Hydrate"** means stoichiometric or nonstoichiometric compositions of compounds or their salts with water.
**"Active component"** (drug-substance) means a physiologically active compound of synthetic or other (biotechnological, vegetable, animal, microbe and so on) origins exhibiting pharmacological activity which is an active ingredient of pharmaceutical composition employing in production and preparation of medicaments.
**"Medicament" -** is a compound (or a mixture of compounds as a pharmaceutical composition) in the form of tablets, capsules, injections, ointments, and other ready forms intended for restoration, improvement or modification of physiological functions in humans and animals, and for treatment and prophylaxis of diseases, for diagnostics, anesthesia, contraception, cosmetology and others.
**"Neuraminidase"** (sialidase, acylneuraminile hydrolase and EC 3.2.1.18) is an enzyme common to animals and to a number of microorganisms. It represents glycohydrolase that cleaves the glycosidic linkages of neuraminic acids in glycoproteins, glycolipids and oligosaccharides. Many of the microorganisms comprising neuraminidase, are pathogenic to humans and such animals as poultry, horses, pigs and seals. These pathogenic organisms include influenza virus. Neuraminidase is associated with influenza virus pathogenicity. Presumably it contributes to elution of newly synthesized virions from infected cells and to virus motion (owing to its hydrolase activity) through respiratory mucous tunic.
**"Pharmaceutical composition"** means a composition comprising a compound of the general formula I and at least one of the components selected from the group consisting of pharmaceutically acceptable and pharmacologically compatible fillers, solvents, diluents, carriers, auxiliary, distributing and excipient agents, delivery agents, such as preservatives, stabilizers, fillers, disintegrators, moisteners, emulsifiers, suspending agents, thickeners, sweeteners, flavoring agents, aromatizing agents, antibacterial agents, fungicides, lubricants, and prolonged delivery controllers, choice and suitable proportions of which depend on the type and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against action of microorganisms can be provided by various antibacterial and antifungal agents, such as, for example, parabens, chlorobutanole, sorbic acid, and similar compounds. Composition may also comprise isotonic agents, such as, for example, sugar, sodium chloride, and similar compounds. Prolonged action of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and injectable organic esters (such as ethyl oleate). Examples of fillers are lactose, milk-sugar, sodium citrate, calcium carbonate, calcium phosphate and the like. Examples of disintegrators and distributors are starch, alginic acid, its salts, and silicates. Examples of suitable lubricants are magnesium stearate, sodium lauryl sulfate, talc and polyethylene glycol of high molecular weight. Pharmaceutical composition for peroral, sublingval, transdermal, intramuscular, intravenous, subcutaneous, local or rectal administration of active ingredient, alone or in combination with another active compound, may be administered to humans and animals in a standard administration form, or in a mixture with traditional pharmaceutical carriers. Suitable standard administration forms include peroral forms such as tablets, gelatin capsules, pills, powders, granules, chewing-gums and peroral solutions or suspensions; sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. These salts could be prepared in situ in the processes of synthesis, isolation or purification of compounds or they could be prepared specially. In particular, salts of bases could be prepared from purified base of the disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, *p*-toluenesulfonates, citrates, maleates, fumarates, succinates, tartrates, methane sulphonates, malonates, salicylates, propionates, ethane sulphonates, benzene sulfonates, sulfamates and the like (Detailed description of such salts properties is given in: Berge S.M., et al., "Pharmaceutical Salts" J.Pharm.Sci., 1977, 66: 1-19). Salts of the disclosed acids may also be prepared by the reaction of purified acids specifically with suitable base; moreover, metal salts and amine salts may be synthesized too. Metal salts are the salts of sodium, potassium, calcium, barium, zinc, magnesium, lithium and aluminum; sodium and potassium salts being preferred. Suitable inorganic bases from which metal salts can be prepared are sodium hydroxide, carbonate, bicarbonate and hydride; potassium hydroxide, carbonate and bicarbonate; lithium hydroxide, calcium hydroxide, magnesium hydroxide, zinc hydroxide. Organic bases suitable for preparation of the disclosed acid salts are amines and amino acids of base strength sufficient to produce stable salt which is suitable for medical purposes use (in particular, they are to be of low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Lysine, ornithine and agrinine can be used as highly basic amino acids.

The inventors have found novel neuraminidase inhibitors representing unknown before fluorosubstituted (3R,4R,5S)-4-fluoroacylamido-5-guanidino-3-(pentan-3-yloxy)cyclohexene-1-carboxylic acids and their esters of the general formula **1** and pharmaceutically acceptable salts thereof, 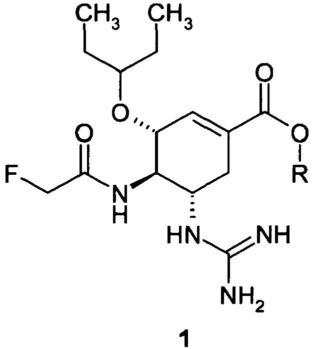 wherein:
**R** represents hydrogen or C₁-C₃ alkyl.

In comparison with the effectiveness of Oseltamivir carboxylate **A2** the novel compounds of the general formula 1, as the inventors have unexpectedly found, are much more effective neuraminidase inhibitors of some other influenza strains by factor of 5-259 (Table 1).

**Table 1. Anti-neuraminidase activity of (3R,4R,5S)-5-guanidino-4-fluoroacetamido-3-(pentan-3-yloxy)cyclohexene-1-carboxylic acid 1.1 and Oseltamivir carboxylate A2 on panel strains of influenza viruses.**

| virus | Inhibitor Nº | | IC^{A2}₅₀ / IC^{1.1}₅₀ |
|---|---|---|---|
| | **A2** | **1.1** | |
| | IC₅₀, nM (number of experiments) | | |
| A/California /07/09 (H1N1) | 0.86 ± 0.42 (8) | 0.18 ± 0.08 (6) | **5** |
| A/Chicken/Rostov on Don /35/07/ (H5N1) | 1.78 ± 0.09 (2) | 0.20 ± 0.00 (2) | **9** |
| A/Pert/261/2009 (H1N1 pdm09; 275Y) | 360 ± 87 (4) | 1.39 ± 0.38 (4) | **259** |
| B/Brisbane/60/2008 | 25.42 ± 0.51 (2) | 1.27 ± 0.14 (2) | **20** |
| B/Pert/211/2001 (197D) | 39.24 ± 2.44 (4) | 2.08 ± 0.71 (4) | **19** |
| B/Pert/211/2001 (197E) | 230.2 ± 62.6 (4) | 6.65 ± 0.90 (4) | **35** |

Novel compounds of the general formula **1,** as the inventors have found, turned out to be more effective inhibitors of neuraminidase of influenza viruses, in comparison with the known **Zanamivir (Zan)** (Table 2).

**Table 2. Anti-neuraminidase activity of (3R,4R,5S)-5-guanidino-4-fluoroacetamido-3-(pentan-3-yloxy)cyclohexene-1-carboxylic acid 1.1 and Zanamivir (Zan) on panel strains of influenza viruses.**

| virus | **inhibitor** | | IC^{Zan}₅₀ / IC^{1.1}₅₀ |
|---|---|---|---|
| | **Zan** | **1.1** | |
| | IC₅₀, nM (number of experiments) | | |
| A/California /07/09 (H1N1) | 0.26 ± 0.32 (6) | 0.18 ± 0.08 (6) | **1.4** |
| A/Aichi/2/69 (H3N2) | 0.60 ± 0.45 (6) | 0.69 ± 0.19 (6) | **0.9** |
| A/Chicken/Rostov on Don/35/07/(H5N1) | 0.59 ± 0.07 (2) | 0.20 ± 0.00 (2) | **3** |
| A/Pert/265/2009 (H1N1; 275H) | 0.46 ± 0.07 (4) | 0.07 ± 0.05 (4) | **6.6** |
| B/Brisbane/60/2008 | 1.87 ± 0.02 (2) | 1.27 ± 0.14 (2) | **1.4** |
| B/Pert/211/2001 (197D) | 3.67 ± 0.20 (4) | 2.08 ± 0.71 (4) | **1.8** |
| B/Pert/211/2001 (197E) | 21.98 ± 5.88 (4) | 6.65 ± 0.90 (4) | **3.2** |

Finally, the inventors found that in comparison with both Oseltamivir carboxylate and Zanamivir the new compounds are more effective in MDCK cell culture towards influenza virus strains la/California/07/09 (H1 N1) and highly pathogenic strain la/duck/MN/1525/81 (H5N1) (Table 3). In particular, (3R,4R,5S)-5-guanidino-4-fluoroacetamido-3-(pentan-3-yloxy)cyclohexene-1-carboxylic acid 1.1 is more active than Oseltamivir carboxylate (**A2**) by factor of 13 and Zanamivir (Zan) by factor of 20 towards la/California/07/09 (H1N1) strain, and towards highly pathogenic strain la/duck/MN/1525/81(H5N1) its activity is higher than that of Oseltamivir carboxylate and Zanamivir (**Zan**) in 24 and 16 times, respectively.

(3R,4R,5S)-5-Guanidino-4-(2-trifluoroacetamido)-3-(pentan-3-yloxy)cyclohexene-1-carboxylic acid **A4** showed neuraminidase activity towards virus A/California/04/09 IC₅₀ = 80 nM, and towards virus A/Vladivostok/16/09 - IC₅₀ > 1000 nM. The unknown before (3R,4R,5S)-5-guanidino-4-fluoroacetamido-3-(pentan-3-yloxy)cyclohexene-1-carboxylic acid **1.1** has neuraminidase activity towards virus A/California/04/09 of 400 times greater than the activity **of A4,** and towards virus A/Vladivostok/16/09 - the novel acid **1.1** has IC₅₀ = 4 nM, that is, it is more active than its known 4-(2-trifluoroacetamido) analogue by factor of 250. (3R,4R,5S)-5-Guanidino-4-(2-difluoroacetamido)-3-(pentan-3-yloxy)cyclohexene-1-carboxylic acid **1.4** (reference example) is also more active than **A4** analogue: its neuraminidase activity towards virus A/California/04/09 corresponds to IC₅₀ = 0.3 nM, (that is, it is 2.7 times more active than **A2** and 267 times more active than **A4);** towards virus A/Vladivostok/16/09 - the novel acid **1.4** exhibits IC₅₀ = 7 nM (in other words, it is more active than **A2** by factor of 118 and by factor of 140 than **A4**).

**Table 3. Specific activity of (3R,4R,5S)-5-guanidino-4-fluoroacetamido-3-(pentan-3-yloxy)cyclohexene-1-carboxylic acid 1.1 in cell culture MDCK towards virus strains la/California/07/09 (H1N1) and highly pathogenic strain Ia/duck/MN/1525/81 (H5N1) in comparison with Oseltamivir carboxylate (A2) and Zanamivir (Zan).**

| **compound** | **Influenza virus strain** | | | |
|---|---|---|---|---|
| | **H1 N1 la/California/07/09** | | **H5N1 la/duck/MN/1525/81** | |
| | **Activity** | | | |
| | **IC₅₀, µM** | **IC₅₀/IC**^{1.1}₅₀ | **IC₅₀, µM** | **IC^{Zan}₅₀ / IC^{1.1}₅₀** |
| **A2** | 9.9 | 16.3 | 77.5 | 24.2 |
| **Zan** | 16.0 | 20.5 | 51.2 | 16.0 |
| **1.1** | **0.78** | | **3.2** | |

According to the present invention the preferred compounds are the compounds of the general formula 1, wherein **R** represents hydrogen, methyl or ethyl.

According to the invention the more preferable compounds of the general formula **1** are: (3R,4R,5S)-5-guanidino-4-fluoroacetamido-3-(pentan-3-yloxy)cyclohexene-1-caboxylic acid **1.1** and its methyl **1.2** and ethyl **1.3** esters.

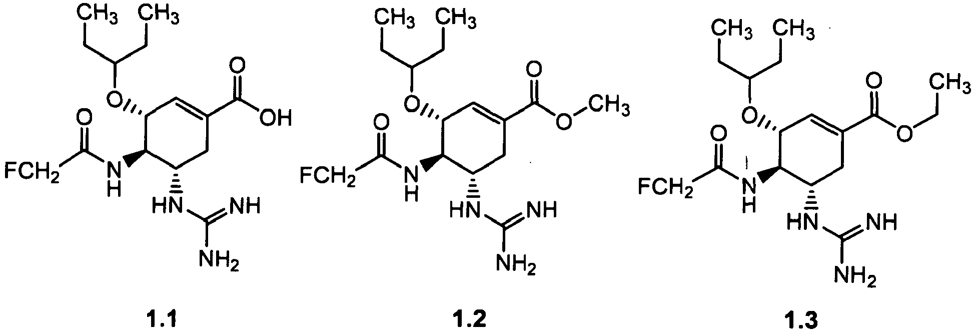

Neuraminidase inhibitors of influenza viruses of the general formula **1** could be prepared starting from alkyl 4-amino-5-(*tert*-butoxycarbonylamino)-3-(pentan-3-yloxy)cyclohexene-1-carboxylate of the general formula **2** according to the scheme shown below:

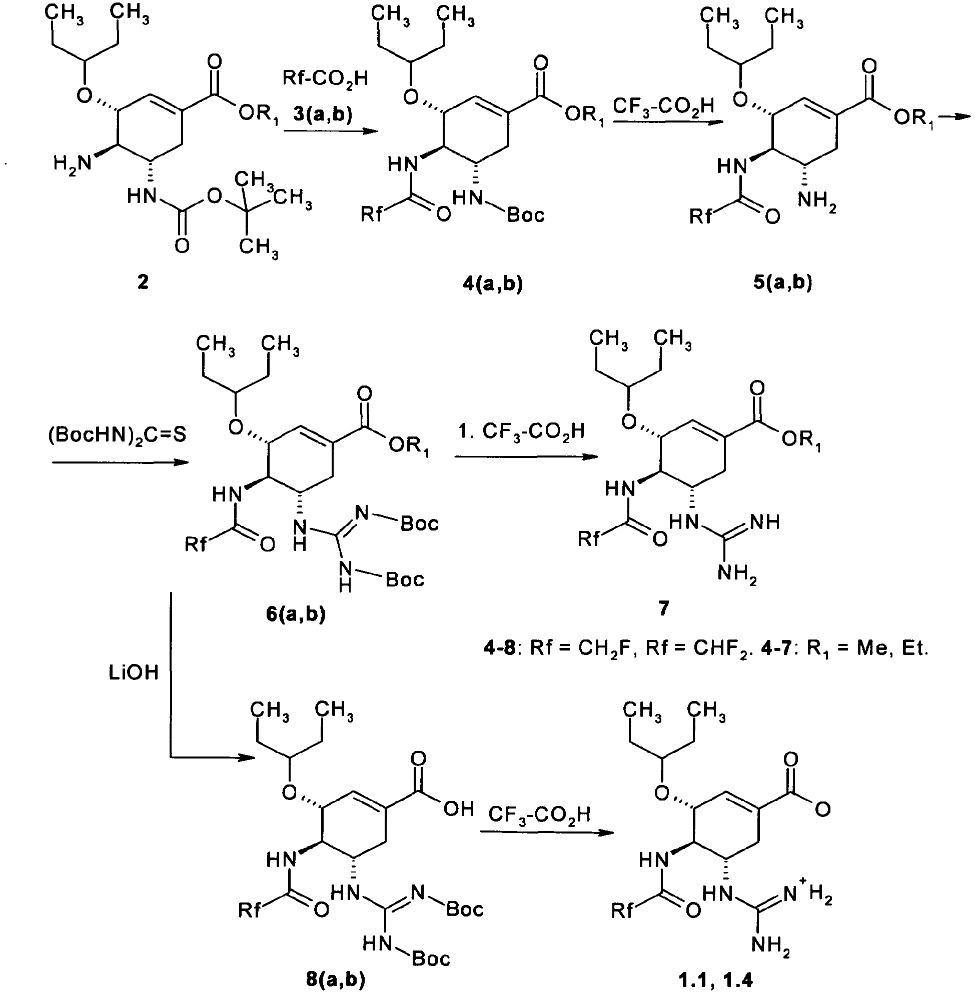

The novel neuraminidase inhibitors of influenza viruses representing unknown before (3R,4R,5S)-4-(2-fluoroacetamido)-5-guanidino-3-(pentan-3-yloxy)cyclohexene-1-carboxylic acids and their esters of the general formula **1** and pharmaceutically acceptable salts thereof also showed high anti-influenza activity in animal models of influenza virus pneumonia.

Anti-neuraminidase activity of the disclosed compounds was determined according to the method described in [Who Collaborating Centre for Reference & Research on Influenza, Australia, Standard Operating Procedure WHO - 025. Reviewed by: Aeron Hurt, Senior Scientist Review Date: 13/3/2009].

According to the present invention novel compounds of the general formula **1** represent an active component for preparation of pharmaceutical compositions and final dosage forms for prophylaxis and treatment of influenza in warm-blooded animals and humans.

The subject of the present invention is a pharmaceutical composition comprising as active component compounds of the general formula **1** or pharmaceutically acceptable salts thereof in therapeutically effective amount.

The pharmaceutical compositions may include pharmaceutically acceptable excipients. Pharmaceutically acceptable excipients mean applied in the sphere of pharmaceutics diluents, auxiliary agents and/or carriers. According to the invention the pharmaceutical composition together with a compound of the general formula **1** or pharmaceutically acceptable salt thereof may involve other active components including those with anti-influenza activity, provided that they do not cause undesirable effects.

If needed, according to the present invention the pharmaceutical composition could be used in clinical practice in various forms prepared by mixing the said compositions with traditional pharmaceutical carries.

According to the present invention the carriers used in pharmaceutical compositions represent the carriers which are used in the sphere of pharmaceutics for preparation of commonly used forms: binding agents, greasing agents, disintegrators, solvents, diluents, stabilizers, suspending agents, colorless agents, taste flavors are used for peroral forms; antiseptic agents, solubilizers, stabilizers are used in forms for injections; base materials, diluents, greasing agents, antiseptic agents are used in local forms.

Pharmaceutical compositions can be prepared by mixing the therapeutically effective amount of at least one active component of the general formula 1 or its pharmaceutically acceptable salt with inert filler and/or solvent.

The subject of the present invention is also a medicament exhibiting anti-influenza activity in the form of tablets, capsules or injections placed in a pharmaceutically acceptable packing and intended for prophylaxis and treatment of influenza in humans and warm-blooded animals and comprising a therapeutically effective amount of the novel active component of the general formula 1 or a pharmaceutical composition comprising the novel active component of the general formula 1.

Therapeutic kits for treating influenza may include as one of the components the novel medicament or the novel pharmaceutical composition comprising as an active component, at least one compound of the general formula 1 or pharmaceutically acceptable salt thereof.

According to the present invention, together with the novel medicament the therapeutic kit for treating influenza may include other known medicaments intended for treatment of influenza or medicaments reinforcing the immune system of patient.

The novel medicament, the novel pharmaceutical composition or the therapeutic kit may be administered to a patient for prophylaxis and treatment of influenza in humans and animals.

The disclosed medicaments could be administered peroral or parenterally (for example, intravenous, subcutaneous, intraperitoneally or local) or by means of inhalator. Clinical dose of an active component of the general formula **1** can be corrected depending on: therapeutic efficiency and bio-accessibility of active ingredients in patients' organism, rate of their exchange and removal from organism, and age, gender, and severity of patient's symptoms. Thus, the daily intake for adults normally is 10∼500 mg, preferably 50∼300 mg. While preparing a medicament as a dose unit from the pharmaceutical composition the above effective dose is to be taken into account, at this, each dose unit of medicament should contain 10∼500 mg, preferably - 50 ∼ 300 mg. Following the instructions of physician or pharmacist, the medicaments may be taken several times over specified periods of time (preferably, from one to six times).

A compound of the general formula 1 may be contacted with neuraminidase for inhibition of neuraminidase activity in vivo, including neuraminidase of influenza virus.

Below the invention is described by means of specific examples which illustrate, but not limit the scope of the invention.
**Example 1** (reference example). **Preparation of ethyl (3R,4R,5S)-4-(2,2-difluoroacetamido)-5-guanidino-3-(pentan-3-yloxy)cyclohexene-1-carboxylate mesylate 1.6·CH₃SO₃H.** 2,2-Difluoroacetic acid **3b** (0.624 g, 0.0065 mol, 1.2 eq) was added dropwise to a solution of ethyl (3*R*,4*R*,5*S*)-4-amino-5-(*tert*-butoxycarbonylamino)-3-(pentan-3-yloxy)cyclohexene-1-carboxylate (2 g, 0.0054 mol, 1 eq.), 1*H-*benzo[d][1,2,3]triazol-1-ol (0.867 g, 0.0065 mol, 1.2 eq.), *N*¹-((ethylimino)methylene)-*N*²,*N*²-dimethylethane-1,2-diamine hydrochloride (1.239 g, 0.0065 mol, 1.2 eq.) and diisopropylethylamine (2.212 g, 0.0178 mol, 3.3 eq.) in THF (20 ml). The reaction mixture was stirred at room temperature for 4 h. Then solvents were evaporated in *vacuo*, the residual oil was dissolved in ethyl acetate, washed with 5% NaHCO₃ solution, dried over Na₂SO₄, filtered and evaporated in *vacuo.* Yield of ethyl (3*R*,4*R*,5*S*)-5-(*tert-*butoxycarbonylamino)-**4**-(2,2-difluoroacetamido)-3-(pentan-3-yloxy)cyclohexene-1-carboxylate **4b** is 89% (2.15 g). LCMS (M+H): found **449;** calculated 448.51. The prepared product **4b** (2.15 g, 0.0048 mol) was dissolved in 10% solution of trifluoroacetic acid in methylene chloride (20 ml) and stirred at room temperature for 12 h. Then solvents were evaporated in *vacuo*, the residual oil was dissolved in ethyl acetate, washed with 5% NaHCO₃ solution, dried over Na₂SO₄, filtered and evaporated in *vacuo.* Yield of the product is 96% (1.605 g). Additional purification was carried out by means of column chromatography with ethyl acetate/THF as eluent, or by recrystallization of the product from hexane. It gives ethyl (3*R*,4*R*,5*S*)-5-amino-4-(2,2-difluoroacetamido)-3-(pentan-3-yloxy)cyclohexene-1-carboxylate **5b,** which was dissolved in methylene chloride and equivalent amount of methanesulfonic acid was added. In 10 min the solvent was evaporated, the obtained product was washed with hexane and dried in *vacuo.* Yield of mesylate **5b·CH₃SO₃H** is 90%. LCMS (M+H): found 349; calculated 348.39. ¹H NMR (DMSO-d₆), 400 MHz: 8.91 (d, *J* = 13.2 Hz, 1 H), 7.83 (br, 3H), 6.74 (s, 1 H), 6.22 (t, *J* = 54 Hz, 1H), 4.31 (d, *J* = 8.4 Hz, 1 H), 4.16 (q, *J* = 7.2 Hz, 2H), 3.45 (m, 1 H), 3.15 (dd, *J*₁ = 11.2 Hz, *J₂* = 8.8 Hz, 1 H), 2.59 (dd, *J₁* = 18 Hz, *J₂* = 6 Hz, 1 H), 2.38 (m, 1 H), 2.31 (s, 3H), 1.66 (m, 1 H), 1.57 (m, 1 H), 1.47 (m, 1 H), 1.39 (m, 1 H), 1.22 (t, *J* = 7.6 Hz, 3H), 0.891 (t, *J* = 7.2 Hz, 3H), 0.842 (t, *J* = 7.2 Hz, 3H). To a solution of ethyl (3R,4R,5S)-5-amino-4-(2,2-difluoroacetamido)-3-(pentan-3-yloxy)cyclohexene-1-carboxylate **5b** (1.6 g; 0.004598 mol; 1eq.) in DMF (16 ml) cooled in ice bath were subsequently added triethylamine (2.57 g; 0.0253 mol; 5.5 eq.), *N*,*N*'-di-boc-thiourea (0.00505 mol; 1.35 g; 1.1eq.) and mercury chloride(II) (0.0055 mol; 1.49 g; 1.2 eq.). The obtained mixture was stirred at cooling in ice bath for 1.5 h. After the reaction was completed the solid was filtered through celite, DMF was evaporated in *vacuo*, the residual oil was dissolved in ethyl acetate, washed with 5% NaHCO₃ solution, dried over Na₂SO₄, filtered and evaporated in *vacuo.* It gives 1.82 g (67%) of ethyl (3*R*,**4***R*,5*S*)-5-((*Z*)-2,3-bis(*tert*-butoxycarbonyl)guanidino)-4-(2,2-difluoroacetamido)-3-(pentan-3-yloxy)cyclohexene-1-carboxylate **6b.** LCMS (M+H): found 591; calculated 590.67. The solution of ethyl (3*R*,4*R*,5*S*)-5-((*Z*)-2,3-bis(*tert-*butoxycarbonyl)guanidino)-**4**-(2,2-difluoroacetamido)-3-(pentan-3-yloxy)cyclohexene-1-carboxylate **6b** (1.82 g; 0.00308 mol) in 10% solution of trifluoroacetic acid in methylene chloride (20 ml) was stirred at room temperature for 12 h. Then the solvents were evaporated in *vacuo*, the residual oil was dissolved in ethyl acetate, washed with 5% NaHCO₃ solution, dried over Na₂SO₄, filtered and evaporated in *vacuo.* It gives 1.1 g (92%) of ethyl (3*R*,4*R*,5*S*)-**4**-(2,2-difluoroacetamido)-5-guanidino-3-(pentan-3-yloxy)cyclohexene-1-carboxylate **1.6.** To a solution of the prepared product in methylene chloride equivalent amount of metanesulfonic acid was added. In 10 min the solvent was evaporated, the product was washed with hexane and dried in *vacuo.* It gives mesylate of ethyl (3*R*,4*R*,5*S*)-4-(2,2-difluoroacetamido)-5-guanidino-3-(pentan-3-yloxy)cyclohexene-1-carboxylate **1.6-CH₃SO₃H,** yield is 95%. LCMS (M+H): found 391; calculated 390.43. ¹H NMR (DMSO-d₆), 400 MHz: 8.67 (d, *J* = 8.4 Hz, 1 H), 7.63 (d, *J* = 9.6 Hz, 1 H), 6.65 (s, 1H), 6.27 (t, *J* = 53.6 Hz, 1H), 4.14 (q, *J* = 7.2 Hz, 2H), 4.12 (m, 7H), 3.54 (m, 1H), 3.41 (m, 1 H), 2.6 (m, 1 H), 2.36 (s, 3H), 1.66 (m, 1 H), 1.44 (m, 4H), 1.22 (t, *J* = 7.2 Hz, 3H), 0.856 (t, *J* = 7.6 Hz, 3H), 0.795 (t, *J* = 7.6 Hz, 3H).
   In a similar way compound **1.7 -** allyl (3R,4R,5S)-5-guanidino-4-(2,2-difluoroacetamido)-3-(pentan-3-yloxy)cyclohexene-1-carboxylate (LCMS (M+H): found 354; calculated 353.44, (towards A/California/04/09 virus IC₅₀ <1 nM) and compound **1.8 -** prop-2-ynyl (3R,4R,5S)-5-guanidino-4-(2,2-difluoroacetamido)-3-(pentan-3-yloxy)cyclohexene-1-carboxylate (LCMS (M+H): found 352; calculated 351.42, (towards A/California/04/09 virus IC₅₀ <1 nM), were prepared when as starting materials (as compound **2**) allyl (3*R*,4*R*,5*S*)-4-amino-5-(*tert*-butoxycarbonylamino)-3-(pentan-3-yloxy)cyclohexene-1-carboxylate and prop-2-ynyl (3*R*,4*R*,5*S*)- -4-amino-5-(*tert-*butoxycarbonylamino)-3-(pentan-3-yloxy)cyclohexene-1-carboxylate were used, respectively.
   Using the corresponding (3*R*,4*R*,5*S*)-4-amino-5-(*tert*-butoxycarbonylamino)-3-(pentan-3-yloxy)cyclohexene-1-carboxylate as a starting material (compound **2**) the following compounds were prepared:
   2-cyclohexyl (3R,4R,5S)-5-guanidino-4-(2,2-difluoroacetamido)-3-(pentan-3-yloxy)cyclohexene-1-carboxylate **1.9** (LCMS (M+H): found 460; calculated 459.55, IC₅₀ <1 nM towards A/California/04/09 virus;
   2-phenylethyl (3R,4R,5S)-5-guanidino-4-(2,2-difluoroacetamido)-3-(pentan-3-yloxy)cyclohexene-1-carboxylate **1.10** (LCMS (M+H): found 454; calculated 453.51, IC₅₀ <1 nM towards A/California/04/09 virus;
   2-pyridin-3-ylethyl (3R,4R,5S)-5-guanidino-4-(2,2-difluoroacetamido)-3-(pentan-3-yloxy)cyclohexene-1-carboxylate **1.11** (LCMS (M+H): found 455; calculated 454.49, IC₅₀ <1 nM towards A/California/04/09 virus);
   2-methoxyethyl (3R,4R,5S)-5-guanidino-4-(2,2-difluoroacetamido)-3-(pentan-3-yloxy)cyclohexene-1-carboxylate **1.12** (LCMS (M+H): found 422; calculated 421.46, IC₅₀ <1 nM towards A/California/04/09 virus).
**Example 2. Mesylate of ethyl (3*R*,4*R*,5*S*)-4-(2-fluoroacetamido)-5-guanidino-3-(pentan-3-yloxy)cyclohexene-1-carboxylate 1.3·CH₃SO₃H** was prepared according to the procedure given in example **1** using monofluoroacetic acid as acylating agent. LCMS (M+H): found 373; calculated 372.44.
**Example 3** (reference example). **(3*R*,4*R*,5*S*)-4-(2,2-difluoroacetamido)-5-guanidino-3-(pentan-3-yloxy)cyclohexene-1-carboxylic acid 1.4. 5%** Solution of lithium hydroxide (2.5 ml) was added to a solution of ethyl (3*R*,4*R*,5*S*)-5-((*Z*)-2,3-bis(*tert*-butoxycarbonyl)guanidino-**4**-(2,2-difluoroacetamido)-3-(pentan-3-yloxy)cyclohexene-1-carboxylate **6b** (250 mg) in dioxane (5 ml) and the reaction mixture was stirred at room temperature for 45 min. Then lithium hydroxide was passivated by adding acetic acid (300 mcl), and the solvents were evaporated in *vacuo.* The obtained solid was extracted with isopropyl alcohol, the extract was dried over Na₂SO₄ and evaporated in *vacuo.* It gives (3*R*,4*R*,5*S*)-5-((*Z*)-2,3-bis(*tert-*butoxycarbonyl)guanidino)-4-(2,2-difluoroacetamido)-3-(pentan-3-yloxy)cyclohexene-1-carboxylic acid **8b** (200 mg, 84%). LCMS (M+H): found 563; calculated 562.62. The obtained acid **8b** (200 mg) was dissolved in 10% solution of trifluoroacetic acid in methylene chloride (2 ml) and stirred at room temperature for 12 h. Then the solvents were evaporated in *vacuo*. (3*R*,4*R*,5*S*)-4-(2,2-Difluoroacetamido)-5-guanidino-3-(pentan-3-yloxy)cyclohexene-1-carboxylic acid **1.4** was separated by HPLC method. LCMS (M+H): found 363; calculated 362.28. ¹H NMR (DMSO-d₆), 400 MHz: 8.68 (d, *J* = 8.4 Hz, 1 H), 7.6 (d, *J* = 10 Hz, 1 H), 7.26 (br, 2H), 6.91 (br, 2H), 6.63 (s, 1 H), 6.27 (t, *J* = 53.6 Hz, 1 H), 4.18 (d, *J* = 8 Hz, 2H), 4.09 (m, 1 H), 3.54 (q, *J* = 10 Hz, 1 H), 3.39 (m, 2H), 2.57 (dd, *J₁* = 18 Hz, *J₂* = 6 Hz, 1 H), 2.31 (m, 1 H), 1.44 (m, 4H), 0.85 (t, *J* = 8 Hz, 3H), 0.795(t, *J* = 7.6 Hz, 3H).
**Example 4. (3*R*,4*R*,5*S*)-4-(2-fluoroacetamido)-5-guanidino-3-(pentan-3-yloxy)cyclohexene-1-carboxylic acid 1.1** was prepared according to the procedure given in example 3. LCMS (M+H): found 345; calculated 345. ¹H NMR (DMSO-d₆), 400 MHz: 8.16 (d, *J* = 10 Hz, 1 H), 7.56 (d, *J* = 9.6 Hz, 1 H), 6.64 (s, 1 H), 4.80 (d, *J* = 47.3 Hz, 2H), 4.21 (d, *J* = 8.4 Hz, 1 H), 3.89 (q, *J* = 10.4 Hz, 1 H), 3.71 (m, 1H), 2.67 (m, 1 H), 2.25 (m, 1 H), 1.42 (m, 4H), 0.85 (t, *J* = 7.2 Hz, 3H), 0.78 (t, *J* = 7.2 Hz, 3H).
**Example 5. Mesylate of methyl (3*R*,4*R*,5*S*)-4-(2-fluoroacetamido)-5-guanidino-3-(pentan-3-yloxy)cyclohexene-1-carboxylate 1.2 CH₃SO₃H** was prepared according to the procedure given in example 1 using methyl (3*R*,4*R*,5*S*)-4-amino-5-(*tert-*butoxycarbonylamino)-3-(pentan-3-yloxy)cyclohexene-1-carboxylate as starting material and monofluoroacetic acid as acylating agent. LCMS (M+H): found 359; calculated 358.42.
**Example 6** (reference example). **Mesylate of methyl (3*R*,4*R*,5*S*)-4-(2,2-difluoroacetamido)-5-guanidino-3-(pentan-3-yloxy)cyclohexene-1-carboxylate 1.5·CH₃SO₃H** was prepared according to the procedure given in example **1** using methyl (3*R*,4*R*,5*S*)-4-amino-5-(*tert*-butoxycarbonylamino)-3-(pentan-3-yloxy)cyclohexene-1-carboxylate as a starting material and difluoroacetic acid as acylating agent. LCMS (M+H): found 373; calculated 372.44.
**Example 7. Preparation of pharmaceutical composition in the form of tablets.** Starch (1600 mg), grained lactose (1600 mg), talcum (400 mg) and (3R,4R,5S)-4-(2-fluoroacetamido)-5-guanidino-3-(pentan-3-yloxy)cyclohexene-1-carboxylic acid **1.1** (1000 mg) were carefully mixed together and pressed into a brick. The prepared brick was crushed into granules and riddled through sieves, gathering granules of 14-16 mesh size. The obtained granules were pelletized into tablets of suitable form of 560 mg by weight each.
**Example 8. Preparation of pharmaceutical composition in capsule form.** (3*R*,4*R*,5*S*)-4-(2-Fluoroacetamido)-5-guanidino-3-(pentan-3-yloxy)cyclohexene-1-carboxylic acid 1.1 was carefully mixed with a powder of lactose in ratio 2:1. The prepared powdery mixture was packed into gelatinous capsules of suitable size by 300 mg each.
**Example 9. Preparation of pharmaceutical composition in injection forms for intramuscular, intraperitoneal or subcutaneous injections.** (3*R*,4*R*,5*S*)-4-(2-Fluoroacetamido)-5-guanidino-3-(pentan-3-yloxy)cyclohexene-1-carboxylic acid 1.1 (500 mg) was mixed with chlorobutanol (300 mg), propylene glycol (2 ml) and injectable water (100 ml). The resultant solution was filtered and placed into 1 ml ampoules which were sealed.
**Example 10. Determination of activity towards neuraminidase of influenza virus for compounds of the general formula 1.** In preliminary experiments working dilutions for strains of allantoic viruses of influenza A/California/07/09 (H1 N1) and A/Aichi/2/69 (H3N2) as well as for resistant influenza virus A/Vladivostok/16/09 (H1 N1) which was prepared in oseltamivir cell culture were determined. For this purpose in 96 - well plate with a round bottom by 60 µl of each virus 2-fold diluted on the reaction buffer mixture (RBM, 50 mM MES, 5 mM CaCl₂, pH 6.5) were prepared. From this plate by 50 µl of 2-fold diluted virus was transferred into 96-well plates with flat bottom for fluorescence measurement (FluoroNunc, black, kat. No. 237105), then, equal volume of substrate buffer (SB, 12.5 mM 2'-(4-methylumbelliferyl)-α-D-N-acetylneuraminic acid, Sigma, 40 mM of acetate buffer pH=5.8) was added to them. As control were used the wells to each of which by 50 µl of RBM was added instead of virus. After incubation of the plate at gentle shaking for 1 h at 37°, 100 µl of stop-solution (2.225 mL of 0.824M NaOH in 11.0mL of ethanol) was added to each well, that was followed by measurement of fluorescence at λₑₓ =360 nm and λₑₘ= 448 nm using Varioskan Flach (Thermo Scientific) instrument. For further experiments were chosen those virus dilutions, which corresponded to the middle of the linear section of the influence curve: virus dilution against value of fluorescence. For determination of anti-neuraminidase activity dilutions of the disclosed compounds and their analogous of formulas **A2** and **A4** (by 50 µl, prepared with RBM) were added to the wells of rows from B to H (used concentrations - 0.03; 0.3; 3; 30; 300; 3000; 30000 nM - each concentration on a row) in 96-well plates with flat bottom for fluorescence measurement (FluoroNunc, black, kat. No. 237105). The wells of A row were used as virus control to which instead of virus the equal volume (50 µl) of RBM was added. Then to the corresponding wells were added by 50 µl of chosen working dilutions (RBM) of each virus. As a control the wells were used to which instead of virus the equal volume of RBM was added. After stirring and incubation at room temperature for 45 min to all wells the equal volume of RBM was added. After repeated stirring and incubation of the plate at 37° for 1 h stop-solution in amount of 100 µl was added to each well. Fluorescence measurements were made at λₑₓ =360 nm and λₑₘ=448 nm with the use of Varioskan Flach (Thermo Scientific) instrument. All determinations were carried out at least in duplicate (two wells of the plate). The percentage of inhibition of neuraminidase activity by tested compound of the general formula 1 was calculated by the formula: inhibition % = 100-(SUF experiment - SUF control / SUF virus control in the absence of compound - SUF control), where SUF is a standard unit of fluorescence). The concentration of a compound at which SUF value was reduced by 50% was taken as inhibitory concentration 50 (IC₅₀).
**Example 11.** Investigation of anti-influenza activity of compounds of the general formula **1** (**1.1., 1.3., 1.4** (reference example) and **1.6** (reference example)) on the model of influenza pneumonia in mice. Pre-weighted mice (female non-linear, average weight 12-15 g) were infected with influenza virus A/Aichi/2/69 (H3N2) (10 LD₅₀ in 50 µl) by means of intranasal introduction under light ether anesthesia. Determination of LD₅₀ was carried out in a preliminary experiment via allantoic virus titration using the same mice that afterwards were used in the main experiment. The following scheme of treatment was used: animals were injected twice: 24 h and 1 h before infection, in 24 h after infection and then 1 time a day during the following 5 days. For oral administration disposable insulin syringe with a special needle (lavage) was used; effect of the following doses was investigated: 25 mg/kg/day of each compound in a volume of 100 µl. As a reference compound was used Tamiflu in doses ranging from 5 mg/kg/day to 30 mg/kg/day. There were 10 animals in the group of "virus control", as well as in each group of animals "treated with compounds" of the general formula **1** or Tamiflu of formula **A3.** The "treated" and control animals were observed daily; during the first 5 days after infection they were weighed every day, further - in a day. Chemotherapeutic activity of compounds in the model of influenza pneumonia in mice was estimated by index of protection against lethal viral infection and by weight loss in the groups of animals treated with the tested compounds in comparison with weight of animals in control group. Lowering or increasing of weight was calculated for each mouse individually and expressed in percentage. The weight of animal before the infection was taken as 100%. For all animals of one group the average percentage of weight loss as well as weight gain was determined.

In a preliminary experiment a dose of virus containing 10 LD₅₀ in volume of 100 µl was determined. Then all animals of the group were infected with this dose. Effectiveness of the compounds of the general formula **1** on the model of influenza pneumonia in mice was estimated by the number of animals survived after virus infection, by average mean lifetime and weight changes of infected animals.

It was found that on the 7-th day of observation all the mice infected with virus and not treated with tested compounds ("virus control" group) died.

The experiments showed that by the time when the last animal of "virus control" group died, death of animals treated with compounds of the general formula 1 and Tamiflu ("treated with compounds") was prevented completely.

Anti-influenza action of the tested compounds of the general formula 1 and Tamiflu of formula **A3** consists in slowing down the weight losses in groups of "treated mice" in comparison with the group of "virus control". Weight loss of animal is one of the clinical implications of influenza pneumonia. More severe course of disease is always accompanied by larger weight loss of the animal. Weighing of mice was carried out on the 1, 2, 3, 4 and 5 days after infection, then every other day up to the 15-th day of observation. It was found that for animals of "virus control" group the most weight loss was observed on the 5-th day after infection (about 10%). In the contrast with the animals of "virus control" group the animals treated with the compounds of the general formula 1 and Tamiflu, on an average, did not lose weight. All the animals of "treated groups" extensively and steadily gained weight starting from the 9^{th} day.

Thus, high efficiency of compounds of formulas **1.1, 1.3, 1.4** (reference example), and 1.6 (reference example) in the treatment of influenza pneumonia in mice has been shown.

### Industrial applicability

The invention can be used in medicine and veterinary.

## Claims

1. (3R,4R,5S)-4-fluoroacylamido-5-guanidino-3-(pentan-3-yloxy)cyclohexene-1-carboxylic acid and their esters of the general formula 1 and pharmaceutically acceptable salts thereof, 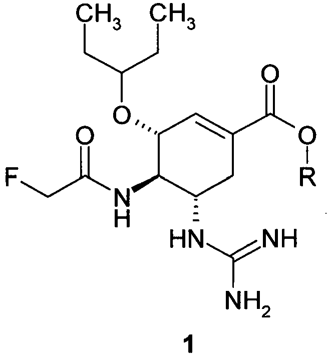 wherein:
**R** represents hydrogen or C₁-C₃ alkyl.

2. The compounds as claimed in claim1 representing:
(3R,4R,5S)-5-guanidino-4-fluoroacetamido-3-(pentan-3-yloxy)cyclohexene-1-carboxylic acid (**1.1**),
methyl (3R,4R,5S)-5-guanidino-4-fluoroacetamido-3-(pentan-3-yloxy)cyclohexene-1-carboxylate (**1.2**),
ethyl (3R,4R,5S)-5-guanidino-4-fluoroacetamido-3-(pentan-3-yloxy)cyclohexene-1-carboxylate (**1.3**).

3. Compounds as defined in claim 1 or 2 for use as a medicament.

4. Compounds for use of claim 3, wherein the medicament exhibits activity towards neuraminidase of influenza virus.

5. A pharmaceutical composition comprising therapeutically effective amount of a compound as claimed in any of claims 1 to 3.

6. The pharmaceutical composition of claim 5, wherein the composition exhibits anti-viral activity.

7. The pharmaceutical composition of claim 6, wherein the composition is intended for treating influenza and concomitant diseases caused by influenza virus.

8. The pharmaceutical composition as claimed in any of claims 5 to 7 in the form of tablets, capsules, or injections placed in a pharmaceutically acceptable packing.

9. The pharmaceutical composition as claimed in any of claims 5 to 8 for treatment of pneumonia caused by influenza virus in effective amount.

10. Compounds as defined in claim 1 or 2 for use in the inhibition of neuraminidase of influenza virus activity.

11. Compounds as defined in claim 1 or 2 for use in the prophylaxis and treatment of influenza and diseases, caused by influenza virus.

## Patentansprüche

1. (3R, 4R, 5S)-4-Fluoracylamido-5-guanidino-3- (pentan-3-yloxy) cyclohexen-1-carbonsäuren und deren Ester der allgemeinen Formel 1 und pharmazeutisch akzeptable Salze davon, 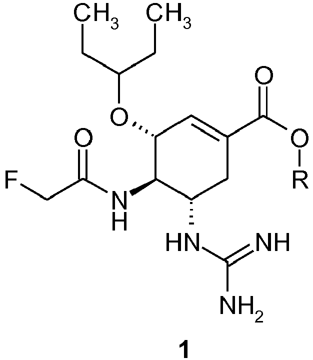 worin
R für Wasserstoff oder C₁-C₃-Alkyl steht.

2. Verbindungen nach Anspruch 1, darstellend:
(3R,4R,5S)-5-Guanidino-4-fluoracetamido-3-(pentan-3-yloxy)cyclohexen-1-carbonsäure (**1.1**),
Methyl-(3R,4R,5S)-5-guanidino-4-fluorcetamido-3-(pentan-3-yloxy)cyclohexen-1-carboxylat (**1.2**),
Ethyl-(3R,4R,5S)-5-guanidino-4-fluoracetamido-3-(pentan-3-yloxy)cyclohexen-1-carboxylate (**1.3**).

3. Verbindungen, wie in Anspruch 1 oder 2 definiert, zur Verwendung als ein Medikament.

4. Verbindungen zur Verwendung nach Anspruch 3, wobei das Medikament eine Aktivität gegenüber der Neuraminidase des Influenzavirus aufweist.

5. Pharmazeutische Zusammensetzung, umfassend eine therapeutisch wirksame Menge einer Verbindung nach jedem der Ansprüche 1 bis 3.

6. Pharmazeutische Zusammensetzung nach Anspruch 5, wobei die Zusammensetzung eine antivirale Aktivität aufweist.

7. Pharmazeutische Zusammensetzung nach Anspruch 6, wobei die Zusammensetzung zur Behandlung von Influenza und Begleiterkrankungen, die durch das Influenza-Virus verursacht sind, bestimmt ist.

8. Pharmazeutische Zusammensetzung nach jedem der Ansprüche 5 bis 7 in Form von Tabletten, Kapseln oder Injektionen, die in einer pharmazeutisch verträglichen Verpackung angeordnet sind.

9. Pharmazeutische Zusammensetzung nach jedem der Ansprüche 5 bis 8 zur Behandlung einer durch das Influenzavirus verursachten Pneumonie in einer wirksamen Menge.

10. Verbindungen, wie in Anspruch 1 oder 2 definiert, zur Verwendung bei der Hemmung von Neuraminidase in der Aktivität des Influenzavirus.

11. Verbindungen, wie in Anspruch 1 oder 2 definiert, zur Verwendung bei der Prophylaxe und der Behandlung von Influenza und Begleiterkrankungen, die durch das Influenza-Virus verursacht sind.

## Revendications

1. Acide (3R,4R,5S)-4-fluoroacylamido-5-guanidino-3-(pentan-3-yloxy)cyclohexène-1-carboxylique et ses esters de la formule générale 1 et ses sels pharmaceutiquement acceptables, 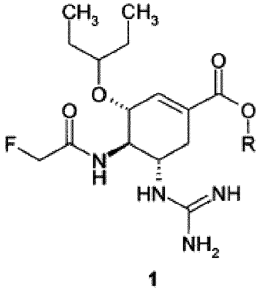 où :
R représente un atome d'hydrogène ou un groupe alkyle en C₁ à C₃.

2. Composés tels que revendiqués selon la revendication 1 représentant :
l'acide (3R,4R,5S)-5-guanidino-4-fluoroacétamido-3-(pentan-3-yloxy)cyclohexène-1-carboxylique (**1.1**),
le (3R,4R,5S)-5-guanidino-4-fluoroacétamido-3-(pentan-3-yloxy)cyclohexène-1-carboxylate de méthyle (**1.2**),
le (3R,4R,5S)-5-guanidino-4-fluoroacétamido-3-(pentan-3-yloxy)cyclohexène-1-carboxylate d'éthyle (**1.3**).

3. Composés tels que définis selon la revendication 1 ou 2 pour l'utilisation comme médicament.

4. Composés pour l'utilisation selon la revendication 3, dans lesquels le médicament fait preuve d'activité vis-à-vis de la neuraminidase du virus de la grippe.

5. Composition pharmaceutique comprenant une quantité thérapeutiquement efficace d'un composé tel que revendiqué selon l'une quelconque des revendications 1 à 3.

6. Composition pharmaceutique selon la revendication 5, la composition faisant preuve d'une activité antivirale.

7. Composition pharmaceutique selon la revendication 6, la composition étant prévue pour traiter la grippe et les maladies concomitantes causées par le virus de la grippe.

8. Composition pharmaceutique telle que revendiquée selon l'une quelconque des revendications 5 à 7 se présentant sous la forme de comprimés, de capsules, ou d'injections placés dans un emballage pharmaceutiquement acceptable.

9. Composition pharmaceutique telle que revendiquée selon l'une quelconque des revendications 5 à 8 pour le traitement de la pneumonie causée par le virus de la grippe en une quantité efficace.

10. Composés tels que définis selon la revendication 1 ou 2 pour l'utilisation dans l'inhibition de l'activité neuraminidase du virus de la grippe.

11. Composés tels que définis selon la revendication 1 ou 2 pour l'utilisation dans la prophylaxie et le traitement de la grippe et des maladies, provoquées par le virus de la grippe.
